# EUROPEAN PATENT SPECIFICATION

(11) **EP 1 164 376 B1**
(45) Date of publication and mention of the grant of the patent: **24.05.2006**
(21) Application number: 00909725.4
(22) Date of filing: 17.03.2000
(51) Int. Cl.: G01N 33/92

(54) **METHOD FOR QUANTITATING CHOLESTEROL**
VERFAHREN ZUR QUANTIFIZIERUNG VON CHOLESTERIN
PROCEDE DE QUANTIFICATION DU CHOLESTEROL

(30) Priority: 24.03.1999 JP 8050399
(43) Date of publication of application: 19.12.2001
(73) Proprietor: DAIICHI PURE CHEMICALS CO., LTD., Chuo-ku Tokyo 103-0027 (JP)
(72) Inventor: YAMAMOTO, Mitsuaki, Daiichi Pure Chemic. Co., Ltd., Ryugasaki-shi, Ibaraki 301-0852 (JP); TAKAHASHI, Yoko, Daiichi Pure Chemicals Co., Ltd., Ryugasaki-shi, Ibaraki 301-0852 (JP); TANIGUCHI, Yuriko, Daiichi Pure Chemic. Co., Ltd., Ryugasaki-shi, Ibaraki 301-0852 (JP); ODAHARA, Shoko, Daiichi Pure Chemicals Co., Ltd., Ryugasaki-shi, Ibaraki 301-0852 (JP); NAKANISHI, Kazuo, Daiichi Pure Chemicals Co., Ltd., Ryugasaki-shi, Ibaraki 301-0852 (JP); NAKAMURA, Mitsuhiro, Daiichi Pure Chem. Co., Ltd., Ryugasaki-shi, Ibaraki 301-0852 (JP); HINO, Koichi, Daiichi Pure Chemicals Co., Ltd., Ryugasaki-shi, Ibaraki 301-0852 (JP)
(74) Representative: HOFFMANN EITLE
(86) International application number: PCT/JP2000/001663
(87) International publication number: WO 2000/057191

(56) References cited:
- EP-A- 0 753 583
- EP-A- 0 754 948
- WO-A1-98/26090
- JP-A- 7 301 636
- JP-A- 9 096 637
- JP-A- 9 121 895
- JP-A- 10 311 833
- JP-A- 11 009 300

## Description

### TECHNICAL FIELD

The present invention relates to a method of quantitative determination of cholesterol which can discriminatively and quantitatively determine the amount of cholesterol present in specific lipoprotein fractions efficiently by a simple procedure using a small amount of sample.

### BACKGROUND ART

Lipids such as cholesterol are combined with an apoprotein and form a lipoprotein in blood serum. Lipoproteins are grouped into chylomicron, very low density lipoprotein (VLDL), low density lipoprotein (LDL), and high density lipoprotein (HDL) according to their physical properties. Among these lipoproteins, LDL is one of the substances which causes arteriosclerosis, whereas HDL is known to exhibit an anti-arteriosclerosis effect.

From the viewpoint of epidemiology, the cholesterol value in LDL has a positive correlation with the frequency of arteriosclerosis occurrence, whereas the cholesterol value in HDL is known to have a reverse correlation with the frequency of arteriosclerosis occurrence. Nowadays, cholesterol values in HDL and LDL are frequently measured for an objective of prevention and diagnosis of ischemic heart diseases. As a method of measuring cholesterols in HDL and LDL, a method of measuring cholesterols after separating HDL and LDL each from other lipoproteins by ultracentrifugation, a method of separating HDL and LDL by electrophoresis, staining lipids, and measuring the intensity of color are known, for example.

However, none of these known methods are used in an everyday practice due to problems such as complicated procedures, incapability of treating a great number of samples, and the like.

A method of measuring cholesterols in HDL widely used in the field of clinical tests is a precipitation method which comprises adding a precipitant to a sample to cause lipoproteins other than HDL to aggregate, removing the aggregate by centrifugation, and measuring cholesterols in the supernatant liquid which contains only HDL. Although this method is simple and easy as compared with ultracentrifugation methods and electrophoretic analysis, the method not only requires a comparatively large amount of sample due to the separating procedure by the addition of a precipitant, but also involves a great risk of analytical error. Therefore, it has been impossible to completely automate the entire analytical process.

A method of discriminating enzymatic determination of cholesterols in HDL has been studied. For example, an enzymatic reaction in the presence of a bile salt and a nonionic surfactant is known (Japanese Patent Application Laid-open No. 126498/1988). This method utilizes the nature of the enzymatic reaction of proceeding in proportion to the LDL cholesterol concentration at the initial stage, but in proportion to the HDL cholesterol concentration at the later stage. However, the analytical accuracy of the method is questioned because it is impossible to completely separate the reaction of cholesterols in HDL and the reaction of cholesterols in other lipoproteins.

Another method known in the art comprises previously aggregating lipoproteins other than HDL, enzymatically reacting only cholesterols in HDL, inactivating the enzyme and, at the same time, redissolving the aggregate, and measuring the absorbance (Japanese Patent Application Laid-open No. 242110/1994). However, this method can be applied only to limited automated analyzers because of requirement of reagent addition at least three times. Thus, the generality of the method is limited. The method is also unsatisfactory from the viewpoint of damage to the analytical instrument and disposal of reagents because redissolution of precipitate requires the use of high concentration of salts and the like.

Japanese Patent No. 2600065 discloses a method of using a precipitation reagent for lipoproteins other than HDL used in a conventional precipitation method and a commonly used reagent for the measurement of cholesterols to measure cholesterols in HDL which have not been precipitated. A specific example disclosed is a combination of a modifying enzyme and α-cyclodextrin sulfate.

The other methods include a method of using a surfactant to reduce the interference of precipitants (Japanese Patent Application Laid-open No. 116996/1996), a method of using antibodys for precipitating lipoproteins other than HDL instead of the conventional precipitation reagent (Japanese Patent Application Laid-open No. 96637/1997, corresponding to EP 0 754 948 Al), a method of using carrageenan (Japanese Patent Application Laid-open No. 121895/1997), and a method of using a sugar compound (Japanese Patent Application Laid-open No. 301636/1995). These methods have problems such as formation of turbidity due to aggregation even in the case where normal serum is mixed, the requirement of aggregating lipoproteins other than HDL (LDL, VLDL, etc.) of which measurement is unnecessary, and the like. As a method of measuring cholesterols in LDL widely accepted in the field of clinical tests, the method of Friedewald (Clinical Chemistry, vol. 18, pp. 459-502 (1972)) is known. According to this method, the amount of LDL cholesterol is determined by using the amounts of total cholesterols, HDL cholesterols, and triglyceride determined by enzymatic methods. This method, however, cannot be applied when the concentration of triglyceride is more than 400 mg/dl.

EP 0 753 583 A1 describes methods for determining cholesterol in high density lipoproteins by using a substance which forms a complex with lipoproteins other than high density lipoproteins and a surfactant, prior to the determination of cholesterol by an enzymatic method.

An object of the present invention, therefore, is to provide a method of quantitative determination of cholesterols in the specific fraction which does not require a pretreatment such as centrifugation, can be carried out efficiently by a simple procedure, and can be applied to various types of automated analyzers.

### DISCLOSURE OF THE INVENTION

As a result of extensive studies, the inventors of the present invention have found that if the reaction of a cholesterol determination enzymatic reagent is carried out in the presence of a compound possessing a stronger affinity with one of the lipoproteins in a sample and of a surfactant exhibiting a stronger action on other lipoproteins in the sample, it is possible to provide a significant difference between the reaction of the cholesterols present in a specific lipoprotein in the sample and the reaction of the cholesterols included in the other lipoproteins, whereby cholesterols in the target lipoprotein can be discriminated and determined with a substantially sufficient accuracy.

Specifically, the present invention provides a method of selectively quantitating cholesterols, comprising determining the amount of cholesterol in a measuring lipoprotein fraction in a sample in the presence of a compound having a stronger affinity with non-measuring lipoproteins in the sample, a surfactant exhibiting a stronger action on the measuring lipoproteins, and a cholesterol determination reagent.

The present invention further provides a method of selectively determining the amount of cholesterol comprising preferentially reacting the cholesterols present in non-measuring lipoproteins in a sample in the presence of a compound having a stronger affinity with the measuring lipoprotein in the sample, a surfactant exhibiting a stronger action on the non-measuring lipoproteins, and a cholesterol determination reagent, and determining the amount of cholesterol in the remaining measuring lipoprotein.

The present invention further provides a method of determining the concentration of cholesterols in various lipoproteins comprising reacting the cholesterols present in a second measuring lipoprotein in a sample in the presence of a compound having a stronger affinity with a first measuring lipoprotein in the sample, a surfactant exhibiting a stronger action on the second lipoprotein than the first lipoprotein, and a cholesterol determination reagent, determining the amount of cholesterol in the remaining first measuring lipoprotein, and determining the concentration of cholesterols in each lipoprotein from the resultant amount of cholesterol in the remaining first measuring lipoprotein and the total cholesterol concentration.

The present invention still further provides a reagent for quantitative determination of cholesterols comprising, as a mixture, a compound having a stronger affinity with one of the lipoproteins in the sample for carrying out the above methods, a surfactant exhibiting a stronger action on the other lipoproteins, and a cholesterol determination reagent.

### BRIEF DESCRIPTION OF THE DRAWINGS

Figure 1 is a chart showing the correlation between the method of Example 4 and a conventional precipitation method.
Figure 2 is a chart showing the correlation between the method of Example 5 and a conventional precipitation method.
Figure 3 is a chart showing the correlation between the method of Example 6 and a conventional precipitation method.
Figure 4 is a chart showing the correlation between the method of Example 7 and a conventional precipitation method.

### BEST MODE FOR CARRYING OUT THE INVENTION

In the present invention, prior to the reaction between cholesterols in lipoproteins and a cholesterol determination reagent, a compound having a stronger affinity with one of the lipoproteins in the sample (hereinafter referred to as "selective affinity agent") and a surfactant exhibiting a stronger action on the other lipoproteins (hereinafter referred to as "selective activator") must be added.

Of these, the selective affinity agent exhibits a mutual action with lipoproteins for which reaction or determination is not desired, and inhibits or suppresses the reaction between those lipoproteins and a cholesterol determination reagent. On the other hand, the selective activator exhibits a strong action on lipoproteins to be reacted or determined when the lipoproteins to be reacted or determined and the lipoproteins for which reaction or determination is not desired are present in the same system, and accelerates the reaction between those lipoproteins to be reacted or determined and the cholesterol determination reagent.

The affinity of the selective affinity agent with one lipoprotein and the action of the selective activator on the other lipoproteins in the present invention must be strong in relative terms, but need not be strong in absolute terms.The reason is because relative errors, which may be significant when only one of them is used, can be reduced to a level at which nQ problem occurs in actual practice when both are used.

As the selective affinity agent used in the present invention, a compound exhibiting affinity with the cholesterols or phospholipids forming the lipoprotein surface layer of the lipoproteins for which reaction or determination is not desired can be given.

As examples of such a selective affinity agent, saponins, polyenes, cholesterol derivatives, peptides, lectins, phospholipid derivatives, and the like can be given. As saponins exhibiting affinity with cholesterols, for example, digitonin, tomatine, and the like can be given;
as polyenes, nystatin, filipin, pimacillyn, pentamycin, trichomycin, fungichromin, perimycin, amphotericin, etoluscomycin, primycin, candigin, and the like can be given;
as cholesterol derivatives, [N-[2-(cholesterylcarboxyaminb)ethyl]carbamoylmethyl]-pullulan (abbreviated to "Chol-AECM-Pullulan") and the like can be given;
as peptides, bacitracin, polymyxin, suzucasylin, gramicidin, and the like can be given;
as lectins, concanavalin A, castor lectin, peanuts lectin, and the like can be given; and
as phospholipid derivatives, L-α-phosphatidyl glycerol dipalmitoyl and the like can be given.

A reaction mixture may become turbid when a sample containing lipoproteins is mixed with some of the above-mentioned selective affinity agents according to the conditions of reagent composition. This may be due to production of aggregate of lipoproteins. However, aggregation of non-measuring lipoproteins is not indispensable in the present invention.

For example, no turbidity is observed when digitonin (a saponin derivative), Chol-AECM-pullulan (a cholesterol derivative), filipin (a polyene compound), L-α-phosphatidyl glycerol dipalmitoyl (a phospholipid derivative), or the like is mixed with a sample containing lipoproteins under conditions in which the effect of the present invention can be exhibited.

It is important for the selective affinity agent of the present invention to adsorb or bind with the components forming lipoprotein surface layers in a manner in which the reaction between cholesterols in lipoproteins and an enzyme is inhibited or suppressed. It is absolutely unnecessary for the lipoproteins to aggregate.

These selective affinity agents may be used either individually or in combination of two or more. Although not specifically limited, the amount of selective affinity agents used differs according to the compounds in the range usually from about 1 nM to 0.1 M (1x10⁻⁷% to 10%), and preferably from 10 nM to 0.1 M (1x10⁻⁶% to 1%). An organic solvent such as alcohol, a surfactant, and a phospholipid can be used to dissolve these compounds. These solvents and the like may be used either individually or in combination of two or more. The amount used differs according to the type of compound to be dissolved, but is not specifically limited.

On the other hand, either an ionic or nonionic selective activator may be used inasmuch as such a selective activator exhibits an action on lipoproteins to be reacted or determined to a different degree in which the selective activator exhibits an action on the lipoproteins for which reaction or determination is not desired. Polyoxyethylene (10) octylphenyl ether, polyoxyethylene higher alcohol ether, polyoxyethylene alkylene phenyl ether, polyoxyethylene alkylene tribenzyl phenyl ether, and the like can be given as examples. Particularly preferable selective activators are polyoxyethylene alkylene phenyl ether and polyoxyethylene alkylene tribenzylphenyl ether, which are known as surfactants exhibiting a strong reactivity with specific lipoproteins when reacted alone with these lipoproteins (Japanese Patent Application Laid-open No. 313200/1997). As examples of commercially available products of these selective activators Triton X-100, Emulgen 709, Emulgen A-60, Emulgen B-66, heptane sulfonic acid, octane sulfonic acid, and the like can be given.

These selective activators may be used either individually or in combinations of two or more. Although not specifically limited, the amount of selective activators used differs according to the compounds in the range from 0.0001%-5%, and preferably from 0.001%-5%.

These selective affinity agents and selective activators can be added to a sample serum either separately or concurrently as a mixture. Any known enzymatic methods can be used for the determination of cholesterols. Examples include a method of using cholesterol esterase and cholesterol oxidase in combination as enzyme reagents, a method of using cholesterol esterase and cholesterol dehydrogenase in combination, and the like. Of these, a method using cholesterol esterase and cholesterol oxidase in combination is preferable.

There are no specific limitations to the method of finally detecting cholesterols after the addition of these cholesterol determination enzyme reagents. For example, an absorbance analysis additionally using a combination of a peroxidase and a coloring substance, a method of directly detecting coenzyme and hydrogen peroxide, and the like can be given.

Preferred specific embodiments of the present invention are as follows:
(1) A method of selectively determining the amount of cholesterol in a measuring lipoprotein in a sample in the presence of a compound having a stronger affinity with non-measuring lipoproteins in the sample, a surfactant exhibiting a stronger action on the measuring lipoproteins, and a cholesterol determination reagent.
(2) A method of selectively determining the amount of cholesterol comprising preferentially reacting the cholesterols present in non-measuring lipoproteins in a sample in the presence of a compound having a stronger affinity with the measuring lipoprotein in the sample, a surfactant exhibiting a stronger action on the non-measuring lipoproteins, and a cholesterol determination reagent, and determining the amount of cholesterol in the remaining measuring lipoprotein.
(3) A method of determining the concentration of cholesterols in various lipoproteins comprising preferentially reacting the cholesterols present in a second measuring lipoprotein in a sample in the presence of a compound having a stronger affinity with a first measuring lipoprotein in the sample, a surfactant exhibiting a stronger action on the second lipoprotein than the first lipoprotein, and a cholesterol determination reagent, determining the amount of cholesterol in the remaining first measuring lipoprotein, and determining the concentration of cholesterols in each lipoprotein from the resultant amount of cholesterol in the remaining first measuring lipoprotein and the total cholesterol concentration.

In practicing the above methods, a reagent for quantitative determination of cholesterols comprising, as a mixture, a selective affinity agent, a selective activator, and a cholesterol determination reagent is used. The reagents for quantitative determination of cholesterols used in the above methods are as follows:
Reagent used in the above method (1):
   A reagent for quantitative determination of cholesterols comprising, as a mixture, a compound having a stronger affinity with non-measuring lipoproteins in the sample, a surfactant exhibiting a stronger action on measuring lipoproteins, and a cholesterol determination reagent.
Reagent used in the above method (2):
   A reagent for quantitative determination of cholesterols comprising, as a mixture, a compound having a stronger affinity with measuring lipoproteins in the sample, a surfactant exhibiting a relatively strong action on non-measuring lipoproteins, and a cholesterol determination reagent.
Reagent used in the above method (3):
   A reagent for quantitative determination of cholesterols comprising, as a mixture, a compound having a stronger affinity with a first measuring lipoprotein in the sample, a surfactant exhibiting a stronger action on a second measuring lipoprotein than on the first measuring lipoprotein, and a cholesterol determination reagent.

A commonly used buffer solution such as a phosphate buffer, a Good's buffer, and the like may be added to the above reagents for the quantitative determination of cholesterols. There are no specific limitations to the pH of a solution in which these reagents for quantitative determination are dissolved to the extent that the enzyme reagents are not affected. In addition, an inorganic salt such as sodium chloride, additives such as albumin used for stabilizing enzyme activity, a salt of divalent metal, a compound acting as a preservative, and the like may also be used.

Cholesterols can be quantitatively determined efficiently by a simple procedure without requiring a pretreatment such as centrifugation by using the method of the present invention described above. In addition, because the method allows specific determination by a simple method using a small amount of sample, the method can be applied to various types of automatic analyzers. The method is thus extremely useful in the field of clinical diagnosis.

### EXAMPLES

The present invention will be described in more detail by examples, which should not be construed as limiting the present invention.

### Example 1 (Reference)

Samples containing lipoproteins were prepared according to the following method given in the preparation of samples. The amount of cholesterol in each lipoprotein fractions was determined by the following method given in the measuring method to compare the reactivity. The results are shown in Table 1.

### (Preparation of samples)

Samples were prepared by fractionating human serum into VLDL, LDL, and HDL by ultracentrifugation.

### (Measuring method)

300 µl of a first reagent, a 50 mM phosphate buffer (pH 6.5) containing 0.005% digitonin, was added to 3 µl of the sample. After 5 minutes, 100 µl of a cholesterol determination reagent (a second reagent), which is a 50 mM phosphate buffer (pH 6.5) containing 0.2% Triton X-100, 1 U/ml cholesterol esterase, 1 U/ml cholesterol oxidase, 5 U/ml peroxidase, 0.04% disulfobutyl-m-toluidine, and 0.004% 4-aminoantipyrine, was added.

Absorbance was measured at 600 nm and 700 nm on the sample immediately before and five minutes after the addition of the cholesterol determination reagent, to compare the difference in the reactivity among the lipoprotein fractions (Two point method) . The above procedure was carried out using Hitachi 7150 automated analyzer (manufactured by Hitachi Ltd.).

### (Results)

**Table 1**

| Sample | No addition of selective affinity agent | Addition of 0.005% digitonin |
|---|---|---|
| HDL fraction | 0.104 (100%) | 0.099 (95%) |
| LDL fraction | 0.315 (100%) | 0.218 (69%) |
| VLDL fraction | 0.267 (100%) | 0.136 (51%) |

| | | |
|---|---|---|
| (a numerical value shows absorbance) | | |

It can be seen from Table 1 that cholesterols in HDL can be preferentially reacted with enzymes over the cholesterols in LDL and the cholesterols in VLDL if digitonin is present.

### Example 2 (Reference)

The amount of cholesterol was determined and compared in the same manner as in Example 1, except that 0.005% Chol-AECM-Pullulan was used instead of digitonin as a first reagent and the surfactant (Triton X-100) in the second reagent was replaced with 1% Emulgen B-66. The results are shown in Table 2.

### (Results)

**Table 2**

| Sample | No addition of selective affinity agent | Addition of 0.005% Chol-AECM-Pullulan |
|---|---|---|
| HDL fraction | 0.133 (100%) | 0.132 (99%) |
| LDL fraction | 0.028 (100%) | 0.019 (67%) |
| VLDL fraction | 0.025 (100%) | 0.013 (50%) |

| | | |
|---|---|---|
| (a numerical value shows absorbance) | | |

It can be seen from Table 2 that cholesterols in HDL can be preferentially reacted with an enzyme over the cholesterols in LDL and the cholesterols in VLDL if Chol-AECM-Pullulan is present.

### Example 3 (Reference)

Samples were prepared in the same manner as in Example 1. The amount of cholesterol in each lipoprotein fractions was determined by the same method as in Example 1 using reagents of the composition shown below to compare the reactivity. The results are shown in Table 3.

### (Reagents used)

### First reagent:

50 mM PIPES buffer solution (pH 6.5) containing 5 mM L-α-phosphatidylglycerol dipalmitoyl and 0.5% Triton X-100

### Cholesterol determination reagent:

50 mM PIPES buffer solution (pH 6.5) containing 1 U/ml cholesterol esterase, 1 U/ml cholesterol oxidase, 5 U/ml peroxidase, 0.04% disulfobutyl-m-toluidine, and 0.004% 4-aminoantipyrine.

### (Results)

**Table 3**

| Sample | No addition of selective affinity agent | Addition of 5 mM L-α-phosphatidylglycerol dipalmitoyl |
|---|---|---|
| HDL fraction | 111.8 (100%) | 107.2 (96%) |
| LDL fraction | 328.6 (100%) | 200.4 (61%) |
| VLDL fraction | 161.9 (100%) | 59.6 (37%) |

| | | |
|---|---|---|
| (a numerical value shows absorbance) | | |

It can be seen from Table 3 that cholesterols in HDL can be preferentially reacted with an enzyme over the cholesterols in LDL and the cholesterols in VLDL if L-α- phosphatidylglycerol dipalmitoyl is present.

### Example 4 (Reference)

Cholesterols in HDL in 25 serum samples containing lipoproteins were determined by the method of the present invention method and the conventional precipitation method, and the measurement value of these methods was compared.

Specifically, 300 µl of a first reagent, a 50 mM MES buffer (pH 6.5) containing 0.005% (40µm) digitonin, was added to 3 µl of the samples. After 5 minutes, 100 µl of a cholesterol determination reagent (a second reagent), which is a 50 mM phosphate buffer (pH 6.5) containing 1% Emulgen B-66, 1 U/ml cholesterol esterase, 1 U/ml cholesterol oxidase, 5 U/ml peroxidase, 0.04% disulfobutyl-m-toluidine, and 0.004% 4-aminoantipyrine, was added.

Absorbance was measured at 600 nm and 700 nm on the sample immediately before and five minutes after the addition of the cholesterol determination reagent, to determine the HDL cholesterol concentration from the difference in the absorbance values (Two point method). A control serum with a known concentration was used as a calibration standard. The above procedure was carried out using Hitachi 7150 automated analyzer (manufactured by Hitachi Ltd.).

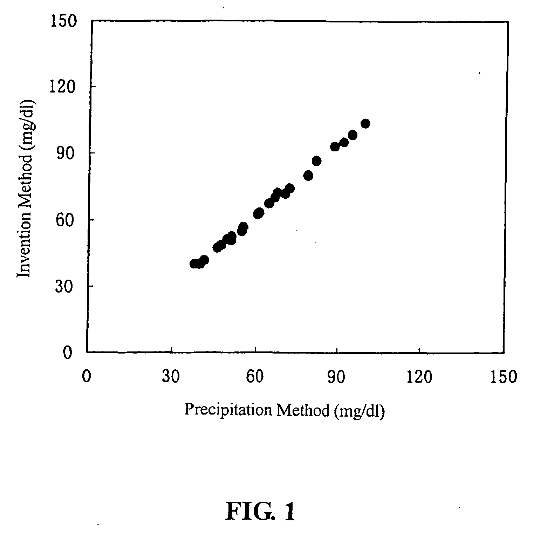

On the other hand, cholesterol determination in HDL by the precipitation method (the comparative method) was carried out as follows. 200 µl of an aqueous solution containing 0.3% of sodium phosphotungstate and 2% of magnesium chloride was mixed with 200 µl of the sample. The mixture was centrifuged at 3000 r.p.m. for 10 minutes. 50 µl of the supernatant solution was mixed with 3 ml of a cholesterol determination reagent, which is a 100 mM MES buffer solution (pH 6.5) containing 1% Triton X-100, 1 U/ml cholesterol esterase, 1 U/ml cholesterol oxidase, 5 U/ml peroxidase, 0.04% disulfobutyl-m-toluidine, and 0.004% 4-aminoantipyrine. The mixture was incubated for 10 minutes at 37°C to measure the absorbance at 600 nm, based on which cholesterols in HDL were determined. The results are shown in Table 4 and Figure 1.

### (Results)

**Table 4**

| Sample No. | Precipitation method (mg/dl) | Invention method (mg/dl) |
|---|---|---|
| 1 | 99.6 | 103.6 |
| 2 | 91.8 | 95.2 |
| 3 | 81.6 | 86.6 |
| 4 | 78.6 | 80.1 |
| 5 | 71.9 | 74.4 |
| 6 | 70.3 | 71.9 |
| 7 | 67.6 | 72.4 |
| 8 | 70.1 | 72.1 |
| 9 | 66.6 | 70.1 |
| 10 | 64.5 | 67.4 |
| 11 | 64.5 | 67.4 |
| 12 | 60.9 | 63.2 |
| 13 | 60.3 | 62.6 |
| 14 | 54.6 | 55.0 |
| 15 | 55.0 | 56.9 |
| 16 | 50.7 | 51.0 |
| 17 | 50.9 | 52.7 |
| 18 | 49.2 | 51.2 |
| 19 | 47.0 | 48.7 |
| 20 | 45.7 | 47.4 |
| 21 | 41.0 | 41.8 |
| 22 | 37.9 | 40.2 |
| 23 | 39.6 | 40.3 |
| 24 | 94.8 | 98.4 |
| 25 | 88.5 | 93.3 |
| Correlation coefficient | | 0.999 0999 |
| Inclination | | 1.052 |
| Intercept | | -0.921 |

As can be seen from Table 4 and Figure 1, the method of Example 4 shows a very good correlation with the conventional precipitation method in spite of the simple procedure.

### Example 5 (Reference)

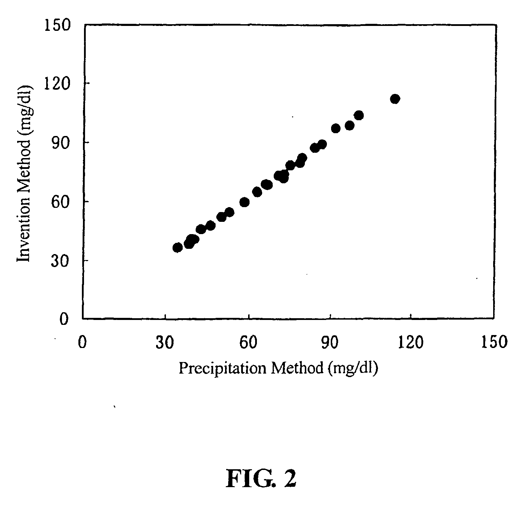

Cholesterols in HDL were determined by the method of Example 4 and the conventional precipitation method using 25 serum samples containing lipoproteins. The method used in Example 4 was followed, except that digitonin in the first reagent was replaced by 0.1 % polymyxin B and 0.005% concanavalin A. The results are shown in Table 5 and Figure 2.

### (Results)

**Table 5**

| Sample No. | Precipitation method (mg/dl) | Invention method (mg/dl) |
|---|---|---|
| 1 | 113.8 | 112.4 |
| 2 | 100.3 | 104.0 |
| 3 | 96.9 | 98.9 |
| 4 | 91.6 | 97.3 |
| 5 | 86.5 | 89.1 |
| 6 | 83.9 | 87.2 |
| 7 | 83.7 | 87.4 |
| 8 | 79.0 | 82.0 |
| 9 | 78.2 | 79.6 |
| 10 | 74.7 | 78.3 |
| 11 | 72.5 | 74.0 |
| 12 | 72.3 | 71.9 |
| 13 | 70.4 | 73.2 |
| 14 | 66.5 | 68.4 |
| 15 | 65.9 | 68.7 |
| 16 | 62.7 | 64.8 |
| 17 | 58.0 | 59.7 |
| 18 | 52.5 | 54.4 |
| 19 | 49.7 | 52.1 |
| 20 | 45.6 | 47.8 |
| 21 | 42.3 | 45.9 |
| 22 | 39.0 | 40.7 |
| 23 | 39.8 | 40.8 |
| 24 | 38.2 | 38.4 |
| 25 | 34.2 | 36.7 |
| Correlation coefficient | | 0.998 |
| Inclination | | 1.007 |
| Intercept | | 1.775 |

As can be seen from Table 5 and Figure 2, the method of Example 5 shows a very good correlation with the conventional precipitation method in spite of the simple procedure.

### Example 6 (Reference)

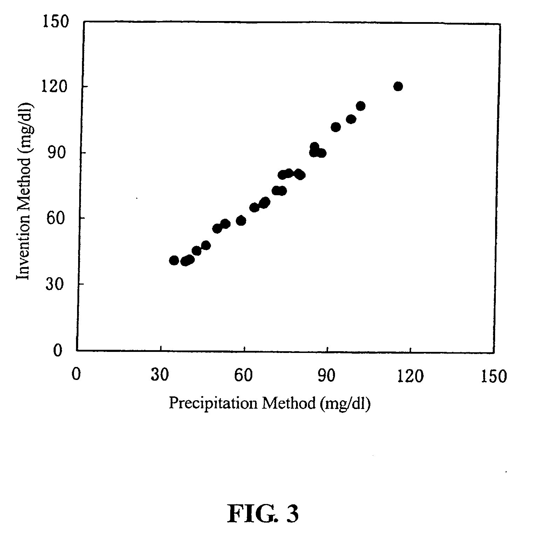

Cholesterols in HDL were determined by the method of Example 4 and the conventional precipitation method using 25 serum samples containing lipoproteins. The method used in Example 4 was followed, except that the digitonin in the first reagent was replaced by 0.005% (76 M) filipin. The results are shown in Table 6 and Figure 3.

### (Results)

**Table 6**

| Sample No. | Precipitation method (mg/dl) | Invention method (mg/dl) |
|---|---|---|
| 1 | 113.8 | 120.7 |
| 2 | 100.3 | 111.7 |
| 3 | 96.9 | 105.6 |
| 4 | 91.6 | 102.0 |
| 5 | 86.5 | 90.2 |
| 6 | 83.9 | 93.0 |
| 7 | 83.7 | 90.5 |
| 8 | 79.0 | 80.0 |
| 9 | 78.2 | 80.7 |
| 10 | 74.7 | 80.9 |
| 11 | 72.5 | 80.1 |
| 12 | 72.3 | 72.9 |
| 13 | 70.4 | 73.1 |
| 14 | 66.5 | 67.9 |
| 15 | 65.9 | 67.0 |
| 16 | 62.7 | 65.3 |
| 17 | 58.0 | 59.4 |
| 18 | 52.5 | 58.0 |
| 19 | 49.7 | 55.8 |
| 20 | 45.6 | 48.0 |
| 21 | 42.3 | 45.6 |
| 22 | 39.0 | 41.2 |
| 23 | 39.8 | 41.7 |
| 24 | 38.2 | 40.7 |
| 25 | 34.2 | 41.1 |
| Correlation coefficient | | 0.993 |
| Inclination | | 1.075 |
| Intercept | | -0.473 |

As can be seen from Table 6 and Figure 3, the method of Example 6 shows a very good correlation with the conventional precipitation method in spite of the simple procedure.

### Example 7

Cholesterols in HDL were determined by the method of the present invention and the conventional precipitation method using 30 serum samples containing lipoproteins.

260 µl of a cholesterol determination reagent containing a selective affinity agent, consisting of a 50 mM PIPES buffer solution (pH 6.5) containing 0.0075% (60 µM) digitonin, 0.25% Emulgen B-66, 0.25 U/ml cholesterol esterase, 0.25 U/ml cholesterol oxidase, 1.25 U/ml peroxidase, 0.01% disulfobutyl-m-toluidine, and 0.005% 4-aminoantipyrine, was added to 2 µl of the sample.

Absorbance was measured at 600 nm and 700 nm on the sample at two minutes and seven minutes after the addition of the cholesterol determination reagent containing a selective affinity agent, to determine the HDL cholesterol from the difference in the absorbance values (Two point method). A control serum with a known concentration was used as a calibration standard. The above procedure was carried out using Hitachi 7170 automated analyzer (manufactured by Hitachi Ltd.).

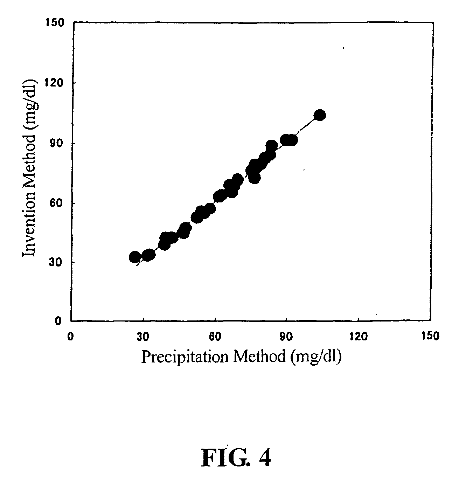

The same method as in Example 3 was used for the cholesterol determination in HDL according to the precipitation method (the comparative method). The results are shown in Table 7 and Figure 4.

### (Results)

**Table 7**

| Sample No. | Precipitation method (mg/dl) | Invention method (mg/dl) |
|---|---|---|
| 1 | 103.2 | 103.7 |
| 2 | 91.8 | 91.6 |
| 3 | 89.4 | 91.6 |
| 4 | 83.4 | 88.6 |
| 5 | 82.7 | 84.2 |
| 6 | 80.6 | 82.3 |
| 7 | 78.9 | 79.7 |
| 8 | 77.0 | 77.6 |
| 9 | 76.2 | 79.1 |
| 10 | 74.9 | 76.4 |
| 11 | 76.1 | 72.9 |
| 12 | 77.6 | 79.4 |
| 13 | 67.5 | 68.6 |
| 14 | 65.7 | 69.0 |
| 15 | 69.0 | 71.9 |
| 16 | 66.5 | 65.4 |
| 17 | 62.3 | 64.0 |
| 18 | 61.3 | 63.2 |
| 19 | 57.3 | 56.8 |
| 20 | 53.9 | 55.4 |
| 21 | 54.9 | 54.5 |
| 22 | 52.1 | 52.4 |
| 23 | 47.4 | 47.1 |
| 24 | 46.4 | 44.6 |
| 25 | 41.7 | 42.3 |
| 26 | 39.1 | 42.1 |
| 27 | 38.6 | 38.6 |
| 28 | 32.3 | 33.5 |
| 29 | 31.8 | 33.2 |
| 30 | 26.5 | 32.4 |
| Correlation coefficient | | 0.995 |
| Inclination | | 0.995 |
| Intercept | | 1.485 |

As can be seen from Table 7 and Figure 4, the method of the present invention shows a very good correlation with the conventional precipitation method in spite of the simple procedure.

## Claims

1. A method of selectively quantifying cholesterols in a sample, comprising determining the amount of cholesterols in a measuring lipoprotein fraction in the sample upon adding a reagent for quantitative determination of cholesterols comprising as a mixture:
- a cholesterol determination reagent,
- a compound having a stronger affinity with the cholesterols or phospholipids that form the lipoprotein surface layer of the non-measuring lipoproteins in the sample than with the cholesterols or phospholipids that form the lipoprotein surface layer of the measuring lipoproteins in the sample, and
- a surfactant exhibiting a stronger action on the measuring lipoproteins than on the non-measuring lipoproteins and promoting reaction with the cholesterol determination reagent.

2. A method of selectively quantifying cholesterols in a sample, comprising determining the amount of cholesterols in a measuring lipoprotein fraction in the sample upon adding a reagent for quantitative determination of cholesterols comprising as a mixture:
- a cholesterol determination reagent,
- at least one compound having a stronger affinity with the non-measuring lipoproteins in the sample than with the measuring lipoproteins in the sample and selected from the group consisting of saponins, polyenes, cholesterol derivatives, lectins, phospholipid derivatives, bacitracin, polymyxin, suzucasylin, and gramicidin, and
- a surfactant exhibiting a stronger action on the measuring lipoproteins than on the non-measuring lipoproteins.

3. The method according to claim 1 or 2, wherein said compound is a saponin.

4. The method according to claim 3, wherein said saponin is a steroid saponin.

5. The method according to claim 3 or 4, wherein said saponin is digitonin or tomatine.

6. The method according to claim 1 or 2, wherein said compound is a polyene.

7. The method according to claim 6, wherein said polyene is an antibiotic.

8. The method according to claim 6 or 7, wherein said polyene is selected from the group consisting of nystatin, filipin, pimacyllin, pentamycin, trichomycin, fungichromin, perimycin, amphotericin, etoluscomycin, primycin, and candigin.

9. The method according to claim 2, wherein said compound is a peptide.

10. The method according to claim 9, wherein said peptide is bacitracin, polymyxin, suzucasylin, or gramicidin.

11. The method according to claim 1 or 2, wherein said compound is a lectin.

12. The method according to claim 11, wherein said lectin is concanavalin A, castor lectin, or peanuts lectin.

13. The method according to claim 1, wherein said compound is a steroid-binding compound.

14. A reagent for the quantitative determination of cholesterols in a sample comprising as a mixture:
- a cholesterol determination reagent,
- a compound having a stronger affinity with the cholesterols or phospholipids that form the lipoprotein surface layer of the non-measuring lipoproteins in the sample than with the cholesterols or phospholipids that form the lipoprotein surface layer of the measuring lipoproteins in the sample,and
- a surfactant exhibiting a stronger action on the measuring lipoproteins than on the non-measuring lipoproteins and promoting reaction with the cholesterol determination reagent.

15. A method of selectively quantifying cholesterols in a sample, comprising
- reacting the cholesterols present in non-measuring lipoproteins in the sample upon adding a reagent for quantitative determination of cholesterols comprising as a mixture:
- a cholesterol determination reagent,
- a compound having a stronger affinity with the cholesterols or phospholipids that form the lipoprotein surface layer of the measuring lipoproteins in the sample than with the cholesterols or phospholipids that form the lipoprotein surface layer of the non-measuring lipoproteins in the sample, and
- a surfactant exhibiting a stronger action on the non-measuring lipoproteins than on the measuring lipoproteins and promoting reaction with the cholesterol determination reagent, and
- determining the amount of cholesterols in the remaining measuring lipoproteins.

16. A reagent for the quantitative determination of cholesterols in a sample comprising as a mixture:
- a cholesterol determination reagent,
- a compound having a stronger affinity with the cholesterols or phospholipids that form the lipoprotein surface layer of the measuring lipoproteins in the sample than with the cholesterols or phospholipids that form the lipoprotein surface layer of the non-measuring lipoproteins in the sample, and
- a surfactant exhibiting a stronger action on the non-measuring lipoproteins than on the measuring lipoproteins and promoting reaction with the cholesterol determination reagent.

17. A method of determining the concentration of cholesterols in each lipoprotein, comprising
- reacting the cholesterols present in second measuring lipoproteins in a sample upon adding a reagent for quantitative determination of cholesterols comprising as a mixture:
- a cholesterol determination reagent,
- a compound having a stronger affinity with the cholesterols or phospholipids that form the lipoprotein surface layer of first measuring lipoproteins in the sample than with the cholesterols or phospholipids that form the lipoprotein surface layer of the second measuring lipoproteins in the sample, and
- a surfactant exhibiting a stronger action on the second measuring lipoproteins than on the first measuring lipoproteins,
- determining the amount of cholesterol in the remaining first measuring lipoproteins, and
- determining the concentration of cholesterols in each lipoprotein from the resultant amount of cholesterols in the remaining first measuring lipoproteins and the total cholesterol concentration.

18. A reagent for the quantitative determination of cholesterols in a sample comprising as a mixture:
- a cholesterol determination reagent,
- a compound having a stronger affinity with the cholesterols and phospholipids that form the lipoprotein surface layer of first measuring lipoproteins in the sample than with the cholesterols and phospholipids that form the lipoprotein surface layer of second measuring lipoproteins in the sample, and
- a surfactant exhibiting a stronger action on the second measuring lipoproteins than on the first measuring lipoproteins.

## Patentansprüche

1. Verfahren zum selektiven Quantifizieren von Cholesterinen in einer Probe, das das Bestimmen der Menge von Cholesterinen in einer zu messenden Lipoproteinfraktion in der Probe nach Zugabe eines Reagens zur quantitativen Bestimmung von Cholesterinen umfasst, das als Mischung umfasst:
• ein Cholesterin-Bestimmungsreagens,
• eine Verbindung mit einer stärkeren Affinität zu den Cholesterinen oder Phospholipiden, die die Lipoprotein-Oberflächenschicht der nicht zu messenden Lipoproteine in der Probe bilden, als zu den Cholesterinen oder Phospholipiden, die die Lipoprotein-Oberflächenschicht der zu messenden Lipoproteine in der Probe bilden, und
• ein Tensid, das eine stärkere Wirkung auf die zu messenden Lipoproteine als auf die nicht zu messenden Lipoproteine ausübt und die Reaktion mit dem Cholesterin-Bestimmungsreagens fördert.

2. Verfahren zum selektiven Quantifizieren von Cholesterinen in einer Probe, das das Bestimmen der Menge von Cholesterinen in einer zu messenden Lipoproteinfraktion in der Probe nach Zugabe eines Reagens zur quantitativen Bestimmung von Cholesterinen umfasst, das als Mischung umfasst:
• ein Cholesterin-Bestimmungsreagens,
• wenigstens eine Verbindung mit einer stärkeren Affinität zu den nicht zu messenden Lipoproteinen in der Probe als zu den zu messenden Lipoproteinen in der Probe, ausgewählt aus der Gruppe, die aus Saponinen, Polyenen, Cholesterinderivaten, Lectinen, Phospholipidderivaten, Bacitracin, Polymyxin, Suzucasylin und Gramicidin besteht, und
• ein Tensid, das eine stärkere Wirkung auf die zu messenden Lipoproteine als auf die nicht zu messenden Lipoproteine ausübt.

3. Verfahren gemäss Anspruch 1 oder 2, worin die Verbindung ein Saponin ist.

4. Verfahren gemäss Anspruch 3, worin das Saponin ein Steroidsaponin ist.

5. Verfahren gemäss Anspruch 3 oder 4, worin das Saponin Digitonin oder Tomatin ist.

6. Verfahren gemäss Anspruch 1 oder 2, worin die Verbindung ein Polyen ist.

7. Verfahren gemäss Anspruch 6, worin das Polyen ein Antibiotikum ist.

8. Verfahren gemäss Anspruch 6 oder 7, worin das Polyen aus der Gruppe ausgewählt ist, die aus Nystatin, Filipin, Pimacyllin, Pentamycin, Trichomycin, Fungichromin, Perimycin, Amphotericin, Etoluscomycin, Primycin und Candigin besteht.

9. Verfahren gemäss Anspruch 2, worin die Verbindung ein Peptid ist.

10. Verfahren gemäss Anspruch 9, worin das Peptid Bacitracin, Polymyxin, Suzucasylin oder Gramicidin ist.

11. Verfahren gemäss Anspruch 1 oder 2, worin die Verbindung ein Lectin ist.

12. Verfahren gemäss Anspruch 11, worin das Lectin Concanavalin A, Rizinus-Lectin oder Erdnuss-Lectin ist.

13. Verfahren gemäss Anspruch 1, worin die Verbindung eine Steroid-bindende Verbindung ist.

14. Reagens zur quantitativen Bestimmung von Cholesterinen in einer Probe, das als Mischung umfasst:
• ein Cholesterin-Bestimmungsreagens,
• eine Verbindung mit einer stärkeren Affinität zu den Cholesterinen oder Phospholipiden, die die Lipoprotein-Oberflächenschicht der nicht zu messenden Lipoproteine in der Probe bilden, als zu den Cholesterinen oder Phospholipiden, die die Lipoprotein-Oberflächenschicht der zu messenden Lipoproteine in der Probe bilden, und
• ein Tensid, das eine stärkere Wirkung auf die zu messenden Lipoproteine als auf die nicht zu messenden Lipoproteine ausübt und die Reaktion mit dem Cholesterin-Bestimmungsreagens fördert.

15. Verfahren zum selektiven Quantifizieren von Cholesterinen in einer Probe, umfassend:
• Umsetzen der in nicht zu messenden Lipoproteinen in der Probe vorhandenen Cholesterine nach Zugabe eines Reagens zur quantitativen Bestimmung von Cholesterinen, das als Mischung umfasst:
- ein Cholesterin-Bestimmungsreagens,
- eine Verbindung mit einer stärkeren Affinität zu den Cholesterinen oder Phospholipiden, die die Lipoprotein-Oberflächenschicht der zu messenden Lipoproteine in der Probe bilden, als zu den Cholesterinen oder Phospholipiden, die die Lipoprotein-Oberflächenschicht der nicht zu messenden Lipoproteine in der Probe bilden, und
- ein Tensid, das eine stärkere Wirkung auf die nicht zu messenden Lipoproteine als auf die zu messenden Lipoproteine ausübt und die Reaktion mit dem Cholesterin-Bestimmungsreagens fördert, und
• Bestimmen der Menge an Cholesterinen in den verbleibenden zu messenden Lipoproteinen.

16. Reagens zur quantitativen Bestimmung von Cholesterinen in einer Probe, das als Mischung umfasst:
• ein Cholesterin-Bestimmungsreagens,
• eine Verbindung mit einer stärkeren Affinität zu den Cholesterinen oder Phospholipiden, die die Lipoprotein-Oberflächenschicht der zu messenden Lipoproteine in der Probe bilden, als zu den Cholesterinen oder Phospholipiden, die die Lipoprotein-Oberflächenschicht der nicht zu messenden Lipoproteine in der Probe bilden, und
• ein Tensid, das eine stärkere Wirkung auf die nicht zu messenden Lipoproteine als auf die zu messenden Lipoproteine ausübt und die Reaktion mit dem Cholesterin-Bestimmungsreagens fördert.

17. Verfahren zur Bestimmung der Konzentration von Cholesterinen in jedem Lipoprotein, umfassend:
• Umsetzen der in zweiten zu messenden Lipoproteinen in einer Probe vorhandenen Cholesterine nach Zugabe eines Reagens zur quantitativen Bestimmung von Cholesterinen, das als Mischung umfasst:
- ein Cholesterin-Bestimmungsreagens,
- eine Verbindung mit einer stärkeren Affinität zu den Cholesterinen oder Phospholipiden, die die Lipoprotein-Oberflächenschicht der ersten zu messenden Lipoproteine in der Probe bilden, als zu den Cholesterinen oder Phospholipiden, die die Lipoprotein-Oberflächenschicht der zweiten zu messenden Lipoproteine in der Probe bilden, und
- ein Tensid, das eine stärkere Wirkung auf die zweiten zu messenden Lipoproteine als auf die ersten zu messenden Lipoproteine ausübt,
• Bestimmen der Menge von Cholesterin in den verbleibenden ersten zu messenden Lipoproteinen und
• Bestimmen der Konzentration von Cholesterinen in jedem Lipoprotein aus der resultierenden Menge von Cholesterinen in den verbleibenden ersten zu messenden Lipoproteinen und der Gesamtcholesterinkonzentration.

18. Reagens zur quantitativen Bestimmung von Cholesterinen in einer Probe, das als Mischung umfasst:
• ein Cholesterin-Bestimmungsreagens,
• eine Verbindung mit einer stärkeren Affinität zu den Cholesterinen oder Phospholipiden, die die Lipoprotein-Oberflächenschicht der ersten zu messenden Lipoproteine in der Probe bilden, als zu den Cholesterinen oder Phospholipiden, die die Lipoprotein-Oberflächenschicht der zweiten zu messenden Lipoproteine in der Probe bilden, und
• ein Tensid, das eine stärkere Wirkung auf die zweiten zu messenden Lipoproteine als auf die ersten zu messenden Lipoproteine ausübt.

## Revendications

1. Procédé de mesure quantitative de manière sélective des cholestérols dans un échantillon, comprenant la détermination de la quantité des cholestérols dans une fraction de lipoprotéines à mesurer dans l'échantillon par addition d'un réactif pour une détermination quantitative des cholestérols comprenant en tant que mélange :
- un réactif de détermination des cholestérols,
- un composé ayant une affinité plus forte avec les cholestérols ou phospholipides qui forment la couche de surface de lipoprotéine des lipoprotéines à ne pas mesurer dans l'échantillon que avec les cholestérols ou phospholipides qui forment la couche de surface de lipoprotéine des lipoprotéines à mesurer dans l'échantillon, et
- un tensioactif présentant une action plus forte sur les lipoprotéines à mesurer que sur les lipoprotéines à ne pas mesurer et favorisant une réaction avec le réactif de détermination des cholestérols.

2. Procédé de mesure quantitative de manière sélective des cholestérols dans un échantillon, comprenant la détermination de la quantité des cholestérols dans une fraction de lipoprotéines à mesurer dans l'échantillon par addition d'un réactif pour une détermination quantitative des cholestérols comprenant en tant que mélange :
- un réactif de détermination des cholestérols,
- au moins un composé ayant une affinité plus forte avec les lipoprotéines à ne pas mesurer dans l'échantillon que avec les lipoprotéines à mesurer dans l'échantillon et choisi dans le groupe constitué par les saponines, les polyènes, les dérivés de cholestérol, les lectines, les dérivés de phospholipide, une bacitracine, une polymyxine, une suzucasyline, et une gramicidine, et
- un tensioactif présentant une action plus forte sur les lipoprotéines à mesurer que sur les lipoprotéines à ne pas mesurer.

3. Procédé selon la revendication 1 ou 2, dans lequel ledit composé est une saponine.

4. Procédé selon la revendication 3, dans lequel ladite saaponine est une saponine de stéroïde.

5. Procédé selon la revendication 3 ou 4, dans lequel ladite saponine est une digitonine ou une tomatine.

6. Procédé selon la revendication 1 ou 2, dans lequel ledit composé est un polyène.

7. Procédé selon la revendication 6, dans lequel ledit polyène est un antibiotique.

8. Procédé selon la revendication 6 ou 7, dans lequel ledit polyène est choisi dans le groupe constitué par une nystatine, une filipine, une pimacylline, une pentamycine, une trichomycine, une fungichromine, une périmycine, une amphotéricine, une étoluscomycine, une primycine et une candigine.

9. Procédé selon la revendication 2, dans lequel ledit composé est un peptide.

10. Procédé selon la revendication 9, dans lequel ledit peptide est une bacitracine, une polymyxine, une suzucasyline ou une gramicidine.

11. Procédé selon la revendication 1 ou 2, dans lequel ledit composé est une lectine.

12. Procédé selon la revendication 11, dans lequel ladite lectine est une concanavaline A, une ricine, ou une lectine d'arachide.

13. Procédé selon la revendication 1, dans lequel ledit composé est un composé se liant aux stéroïdes.

14. Réactif pour la détermination quantitative des cholestérols dans un échantillon comprenant en tant que mélange :
- un réactif de détermination des cholestérols,
- un composé ayant une affinité plus forte avec les cholestérols ou phospholipides qui forment la couche de surface de lipoprotéine des lipoprotéines à ne pas mesurer dans l'échantillon que avec les cholestérols ou phospholipides qui forment la couche de surface de lipoprotéine des lipoprotéines à mesurer dans l'échantillon, et
- un tensioactif présentant une action plus forte sur les lipoprotéines à mesurer que sur les lipoprotéines à ne pas mesurer et favorisant une réaction avec le réactif de détermination des cholestérols.

15. Procédé de mesure quantitative de manière sélective des cholestérols dans un échantillon, comprenant
- la réaction des cholestérols présents dans des lipoprotéines à ne pas mesurer dans l'échantillon par addition d'un réactif pour une détermination quantitative des cholestérols comprenant en tant que mélange :
- un réactif de détermination des cholestérols,
- un composé ayant une affinité plus forte avec les cholestérols ou phospholipides qui forment la couche de surface de lipoprotéine des lipoprotéines à mesurer dans l'échantillon que avec les cholestérols ou phospholipides qui forment la couche de surface de lipoprotéine des lipoprotéines à ne pas mesurer dans l'échantillon, et
- un tensioactif présentant une action plus forte sur les lipoprotéines à ne pas mesurer que sur les lipoprotéines à mesurer et favorisant une réaction avec le réactif de détermination des cholestérols, et
- la détermination de la quantité des cholestérols dans les lipoprotéines à mesurer restantes.

16. Réactif pour la détermination quantitative des cholestérols dans un échantillon comprenant en tant que mélange :
- un réactif de détermination des cholestérols,
- un composé ayant une affinité plus forte avec les cholestérols ou phospholipides qui forment la couche de surface de lipoprotéine des lipoprotéines à mesurer dans l'échantillon que avec les cholestérols ou phospholipides qui forment la couche de surface de lipoprotéine des lipoprotéines à ne pas mesurer dans l'échantillon, et
- un tensioactif présentant une action plus forte sur les lipoprotéines à ne pas mesurer que sur les lipoprotéines à mesurer et favorisant une réaction avec le réactif de détermination des cholestérols.

17. Procédé de détermination de la concentration des cholestérols dans chaque lipoprotéine comprenant
- la réaction des cholestérols présents dans des secondes lipoprotéines à mesurer dans un échantillon par addition d'un réactif pour la détermination quantitative des cholestérols comprenant en tant que mélange :
- un réactif de détermination des cholestérols,
- un composé ayant une affinité plus forte avec les cholestérols ou phospholipides qui forment la couche de surface de lipoprotéine des premières lipoprotéines à mesurer dans l'échantillon que avec les cholestérols ou phospholipides qui forment la couche de surface de lipoprotéine des secondes lipoprotéines à mesurer dans l'échantillon, et
- un tensioactif présentant une action plus forte sur les seconde lipoprotéines à mesurer que sur les premières lipoprotéines à mesurer,
- la détermination de la quantité de cholestérol dans les premières lipoprotéines à mesurer restantes, et
- la détermination de la concentration des cholestérols dans chaque lipoprotéine à partir de la quantité résultante des cholestérols dans les premières lipoprotéines à mesurer restantes et la concentration totale des cholestérols.

18. Réactif pour la détermination quantitative des cholestérols dans un échantillon comprenant en tant que mélange :
- un réactif de détermination des cholestérols,
- un composé ayant une affinité plus forte avec les cholestérols ou phospholipides qui forment la couche de surface de lipoprotéine des premières lipoprotéines à mesurer dans l'échantillon que avec les cholestérols ou phospholipides qui forment la couche de surface de lipoprotéine des secondes lipoprotéines à mesurer dans l'échantillon, et
- un tensioactif présentant une action plus forte sur les secondes lipoprotéines à mesurer que sur les premières lipoprotéines à mesurer.
